# EUROPÄISCHE PATENTANMELDUNG

(11) **EP 4 567 830 A1**
(43) Veröffentlichungstag der Anmeldung: **11.06.2025**
(21) Anmeldenummer: 23214507.8
(22) Anmeldetag: 06.12.2023
(51) Int. Cl.: G16H 40/63, G16H 40/67

(54) **VERFAHREN UND VORRICHTUNG ZUM ANPASSEN EINES OPERATIONELLEN ARBEITSPLATZES AN EINEN OPERATEUR**

(71) Anmelder: Siemens Healthineers AG, 91301 Forchheim (DE)
(72) Erfinder: Brandt, Jan-Mels, 91074 Herzogenaurach (DE)
(74) Vertreter: Siemens Healthineers Patent Attorneys

(57) **Zusammenfassung**

Die Erfindung betrifft ein Verfahren zur Konfiguration eines interventionellen Therapieplatzes an einen Operateur zur Durchführung eines Eingriffs. Der Therapieplatz weist eine Steuerung, eine Schnittstelle zur Eingabe einer Information über den Eingriff, eine Vorrichtung zum Erfassen eines Operateurs und mindestens eine Ausgabeeinheit zur Anpassung der Konfiguration des Therapieplatzes in Abhängigkeit von dem Eingrifft und der Eigenschaft des Operateurs auf. Die Erfindung betrifft weiterhin eine medizinische Bildgebungsvorrichtung zur Ausführung des erfindungsgemäßen Verfahrens auf sowie ein Verfahren zum Trainieren eines neuronalen Netzwerkes der Steuerung.

## Beschreibung

*Unabhängig vom grammatikalischen Geschlecht eines bestimmten Begriffes sind Personen mit männlicher, weiblicher oder anderer Geschlechteridentität mit umfasst.*

Die Erfindung betrifft ein Verfahren zur Konfiguration eines interventionellen Therapieplatzes zur Durchführung eines Eingriffs. Der Therapieplatz weist eine Steuerung, eine Schnittstelle zur Eingabe einer Information über den Eingriff, eine Vorrichtung zum Erfassen eines Operateurs und mindestens eine Ausgabeeinheit auf. Die Erfindung betrifft weiterhin eine medizinische Bildgebungsvorrichtung zur Ausführung des erfindungsgemäßen Verfahrens auf sowie ein Verfahren zum Trainieren eines neuronalen Netzwerkes der Steuerung.

Im medizinischen Bereich sind an einer Intervention an einem Patienten neben dem Patienten und dem ausführenden Operateur immer ein oder mehrere Vorrichtungen beteiligt. Dies kann eine Vorrichtung zur Lagerung des Patienten wie beispielsweise ein Patiententisch oder eine Patientenliege sein. Falls der Operateur nicht steht, kann für ihn eine Sitzgelegenheit vorgesehen sein. Selbst wenn der Operateur stehend arbeitet, können unterschiedliche Körperhaltungen zum Ausführen der Intervention eingenommen werden. An der Intervention können medizinische Instrumente beteiligt sein, die von Haltevorrichtungen ausgerichtet oder geführt werden. Nicht zuletzt kann eine bildgebende Vorrichtung zur Überwachung des Eingriffs vorgesehen sein, beispielsweise ein C-Bogen oder ein Magnetresonanztomograph, insbesondere dann, wenn der Eingriff bzw. die Intervention im Inneren des Patienten erfolgt, z.B. minimal-invasiv.

Der Erfolg des Eingriffs bzw. der Eingriffe im Lauf eines Arbeitstages hängt dabei von vielen Faktoren ab. Insbesondere das Zusammenspiel des Operateurs und der einzelnen Komponenten sichert dabei einen andauernden Erfolg.

Aus der Automobilindustrie ist es bekannt, einen Fahrer anhand eines individuellen Schlüssels zu identifizieren und Lenkrad- sowie Sitzposition an die letzten Einstellungen des Fahrers anzupassen.

Es ist eine Aufgabe der Erfindung, einen interventionellen Therapieplatz bereitzustellen, der eine kontinuierliche Qualität für Eingriffe verbessert.

Die Aufgabe wird durch ein erfindungsgemäßes Verfahren zur Konfiguration eines interventionellen Therapieplatzes nach Anspruch 1, sowie eine erfindungsgemäße medizinische Bildgebungsvorrichtung nach Anspruch 8 und ein erfindungsgemäßes Verfahren zum Training eines neuronalen Netzwerkes nach Anspruch 9 gelöst.

Das erfindungsgemäße Verfahren ist zur Konfiguration eines interventionellen Therapieplatzes an einen Operateur zur Durchführung eines Eingriffs vorgesehen.

Als interventioneller bzw. interoperationeller Therapieplatz wird im Sinne der Erfindung ein System aus Vorrichtungen angesehen, die bei einem Eingriff am Patienten benötigt werden, beispielsweise eine Vorrichtung wie eine Patientenliege zur Lagerung des Patienten, kann aber auch eine Sitzgelegenheit für den Operateur, ein medizinisches (medical device) Instrument zur Durchführung des Eingriffs oder eine medizinische Bildgebungsvorrichtung zur Überwachung des Eingriffs umfassen. Als Intervention wird jeder medizinischer Eingriff in den Patienten durch den Operateur angesehen, wie zum Beispiel eine Nadelbiopsie, ein minimalinvasiver Eingriff, ein Eingriff über einen Katheter, eine Ablation oder auch ein klassischer chirurgischer Eingriff.

Als Konfiguration wird dabei eine Summe an Eigenschaften des Therapieplatzes angesehen, die zum einen veränderbar sind, zum anderen einen Einfluss auf eine Ausführung eines Eingriffs haben. Die Konfiguration im erweiterten Sinn der Erfindung kann auch die an einem Eingriff beteiligten Personen wie Operateur und Patient umfassen. Die Konfiguration betrifft insbesondere eine Anordnung der einzelnen an einem Eingriff beteiligten Gegenstände und Personen relativ zueinander. Für Gegenstände wie Sitzgelegenheit, Instrumente oder Bildgebungsvorrichtung kann diese beispielsweise durch Stellglieder veränderlich sein. Für Personen ist dies beispielsweise durch verfahrbare Patiententische oder Sitzgelegenheiten realisierbar, denkbar ist aber auch eine Anzeige oder sonstige Ausgabe, die Vorgaben an die Personen zur Positionierung ausgibt. Denkbar ist bei der Konfiguration auch eine zeitliche Komponente, d.h. die Konfiguration kann sich während eines Eingriffs verändern und die veränderte Konfiguration durch die Ausgabeeinheit ausgegeben werden, kontinuierlich oder zu vorbestimmten Zeitpunkten.

Der interventionelle Therapieplatz weist eine Steuerung auf. Unter Steuerung wird ein Prozessor bzw. Computer verstanden, der von mindestens einer Funktionseinheit des Therapieplatzes eine Information empfängt und mindestens eine Vorrichtung des Therapieplatzes steuert. Die Steuerung kann lokal in einem Raum gemeinsam mit den anderen Komponenten des Therapieplatzes sein, aber auch räumlich abgesetzt und/oder in einer Cloud oder einem Datencenter über ein Datennetzwerk realisiert sein.

Die Steuerung weist eine Schnittstelle zur Eingabe einer Information über den Eingriff auf. Die Schnittstelle kann beispielsweise eine Bedienerschnittstelle wie eine GUI sein, oder auch eine Datenschnittstelle zu einem Verwaltungssystem einer Klinik oder Praxis. Vorzugsweise identifiziert die Information die Art des Eingriffs, insbesondere auf die bei dem Eingriff verwendeten Vorrichtungen des Therapieplatzes und deren relative Position zueinander.

Der Therapieplatz weist weiterhin eine Vorrichtung zum Erfassen eines Operateurs auf. Unter Erfassen des Operateurs wird das Erfassen einer Information verstanden, die zumindest eine physiologische Eigenschaft des Operateurs identifiziert. Dies kann indirekt erfolgen, indem die Identität des Operateurs durch die Information bestimmt ist, beispielsweise über eine Kamera mit Gesichtserkennung oder durch ein Token wie mit RFID, und über die Information Zugriff auf mindestens eine gespeicherte physiologische Eigenschaft des Operateurs besteht, beispielsweise in einer Datenbank. Es ist aber auch denkbar, dass die physiologische Eigenschaft unmittelbar erfasst wird, beispielsweise durch eine 3D-Kamera und/oder eine kinematische Auswertung einer Videoaufnahme mit der 3D-Kamera oder einer 2D-Kamera.

Denkbar ist auch ein Marker oder Sensor am Körper des Operateurs, beispielsweise eine biometrische Jacke bzw. Kleidungsstück oder auch Tracking-Points am Körper.

Weiterhin weist der Therapieplatz mindestens eine Ausgabeeinheit zur Anpassung der Konfiguration des Therapieplatzes auf. Die Ausgabeeinheit kann eine Textausgabe sein oder auch eine graphische Ausgabe. Vorzugsweise handelt es sich aber, wie nachfolgend zu einem Unteranspruch ausgeführt, um ein Stellglied, mit dem eine Konfiguration des Therapieplatzes unmittelbar ausgeführt werden kann.

In einem Schritt des Verfahrens wird zunächst eine Eigenschaft des Operateurs mit der Vorrichtung zum Erfassen ermittelt. Dies kann wie bereits beschrieben unmittelbar das Erfassen einer physiologischen Eigenschaft z.B. durch eine 3D- oder 2D-Kamera sein. Auch können kinematische Daten mittels der Kamera ermittelt werden. Denkbar ist aber auch ein mittelbares Erfassen durch Erfassen einer Identifikation der Person des Operateurs und dem Abrufen mindestens einer physiologischen Eigenschaft anhand der Identifikation aus einem Speicher oder einer Datenbank.

In einem weiteren Schritt wird, wie bereits beschrieben, von der Steuerung über die Schnittstelle der vorzunehmende Eingriff ermittelt. Darunter sind zumindest Informationen zu verstehen, die den Eingriff in Bezug auf eine Konfiguration des Therapieplatzes dafür betreffen, beispielsweise Position und relative Ausrichtung von Patienten, Bildgebungsvorrichtung und/oder Operateur zueinander.

In einem weiteren Schritt wird eine Konfiguration für den interventionellen Therapieplatz in Abhängigkeit von dem vorzunehmenden Eingriff und mindestens einer Eigenschaft des Operateurs durch die Steuerung ermittelt. Beispielsweise kann anhand der Körpergröße, Anatomie, Geschlecht, Armlänge etc. und des vorzunehmenden Eingriffs eine generelle Position des Operateurs relativ zu dem Patiententisch in Richtung dessen x, y und z-Achse ermittelt werden, bei der der Operateur sich weder zu tief bücken noch zu weit strecken muss, um den Ort am Patienten für den Eingriff zu erreichen und so einer schnellen Ermüdung entgehen kann. Auch kann die Position einer Bildgebungsvorrichtung oder eines medizinischen Instruments betroffen sein, die dem Operateur nicht im Weg sein dürfen und/oder in dessen Reichweite sein müssen.

Anschließend wird die ermittelte Konfiguration über die Ausgabeeinheit ausgegeben, beispielsweise in Textform oder durch eine grafische Darstellung auf einem Display, oder, wie nachfolgend zu den Unteransprüchen ausgeführt, durch direktes Einstellen von einem oder mehreren Stellgliedern des Therapieplatzes. Betrifft die Konfiguration insbesondere eine beteiligte Person, z.B. den Patienten oder insbesondere den Operateur, kann die Ausgabeeinheit eine graphische, textliche oder auch akustische Wiedergabemöglichkeit in Tönen bzw. Sprache aufweisen. Insbesondere, wenn die Konfiguration des Therapieplatzes eine Haltung oder Position des Operateurs betrifft, ist es auch denkbar, dass die Steuerung die Position des Operateurs erfasst, z.B. mittels der Kamera, und mit der ermittelten Konfiguration, d.h. der Position und/oder Haltung vergleicht, eine Abweichung ermittelt und über die Ausgabeeinheit ausgibt. Denkbar sind auch Ausgaben zur Korrektur hin zur Sollposition.

Auf vorteilhafte Weise ermöglicht das erfindungsgemäße Verfahren durch eine vorteilhafte Konfiguration des Therapieplatzes für den jeweiligen Eingriff und Operateur eine ergonomisch vorteilhafte und biomechanisch optimale Position einzunehmen und die Tätigkeit mit geringerer Ermüdung auszuführen.

Die erfindungsgemäße medizinische Bildgebungsvorrichtung weist eine Steuerung mit einer Schnittstelle zur Eingabe einer Information über einen Eingriff auf. Wie bereits zu dem Verfahren erläutert, kann es sich dabei um eine Bedienschnittstelle für einen Nutzer bzw. Operateur handeln, aber auch um eine Datenschnittstelle, beispielsweise zu einem Verwaltungssystem für Patientendaten.

Die erfindungsgemäße medizinische Bildgebungsvorrichtung weist weiterhin eine Vorrichtung zum Erfassen eines Operateurs auf. Wie vorangehend erläutert, kann es sich dabei um eine Vorrichtung handeln, um eine Identität des Operateurs zu erfassen, um anschließend physiologische Informationen zu dem Operateur aus anderer Quelle zu beziehen, wie z.B. einer Datenbank. Als Vorrichtung sind z.B. eine Kamera mit Gesichtserkennung oder auch ein RFID-Empfänger für ein Token des Operateurs möglich. Ebenfalls denkbar ist aber auch eine direkte Erfassung von physiologischen Eigenschaften des Operateurs, beispielsweise mit einer Videokamera oder einer 3D-Kamera. Es ist aber auch denkbar, dass eine physiologische Eigenschaft des Operateurs mit der medizinischen Bildgebungsvorrichtung selbst erfasst wird.

Weiterhin weist die erfindungsgemäße Bildgebungsvorrichtung mindestens eine Ausgabeeinheit zur Anpassung einer Konfiguration des Therapieplatzes auf. Dies kann beispielsweise ein Display zur Ausgabe einer Konfiguration an einen Nutzer sein, in der die einzunehmende Konfiguration wiedergegeben ist oder Hinweise, wie diese zu erreichen ist. Insbesondere kann aber die Ausgabevorrichtung auch mindestens ein Stellglied aufweisen, mit dem eine Konfiguration der medizinischen Bildgebungsvorrichtung verändert werden kann. Das Stellglied kann beispielsweise eine Höhen- oder Positionsverstellung des Patiententischs oder eines Elements zur Bilderfassung sein, wie beispielsweise ein C-Bogen.

Insbesondere ist die erfindungsgemäße Bildgebungsvorrichtung ausgebildet, mit ihrer Steuerung das erfindungsgemäße Verfahren auszuführen, indem sie beispielsweise in ihrer Steuerung ein gespeichertes Programm mit Anweisungen zur Durchführung des Verfahrens ausführt.

Die erfindungsgemäße medizinische Bildgebungsvorrichtung teilt die Vorteile des erfindungsgemäßen Verfahrens.

Die Erfindung betrifft weiterhin ein Verfahren zum Trainieren eines neuronalen Netzwerkes, das bei einer Ausführung des erfindungsgemäßen Verfahrens verwendet wird. Das neuronale Netzwerk kann Teil der Steuerung der medizinischen Bilderfassungsvorrichtung sein oder kann auch in einer anderen Komponente des interventionellen Therapieplatzes vorgesehen sein. Denkbar ist auch eine Lokalisierung des neuronalen Netzwerkes in einem abgesetzten Server oder einem Clouddienst. Das neuronale Netzwerk ist insbesondere vorgesehen, den Schritt Ermitteln einer Konfiguration für den interventionellen Therapieplatz in Abhängigkeit von dem vorzunehmenden Eingriff und mindestens einer Eigenschaft des Operateurs auszuführen oder zumindest zu unterstützen.

In einem Schritt des erfindungsgemäßen Verfahrens zum Trainieren werden eine Vielzahl von Tupeln aus Eigenschaften von Operateuren, von Eingriffen und von zugeordneten Konfigurationen als Trainingsdaten bereitgestellt. Mit anderen Worten, es werden beispielsweise in der Vergangenheit protokollierte Eingriffe, dabei erfasste oder anhand der Identität zugeordnete Eigenschaften des Operateurs und verwendete Konfigurationen des Therapieplatzes auf einem Datenträger oder in einem Speicher bereitgestellt. Es ist auch denkbar, dass derartige Tupel künstlich erzeugt werden, z.B. durch Versuchspersonen an einem vergleichbaren Therapieplatz oder durch entsprechende digitale Zwillinge unter Verwendung von kinematischen Programmen bzw. Programmen zur Bewertung der Ergonomie. Die Daten könne entweder lokal oder in der Cloud gespeichert werden.

In einem weiteren Schritt wird das neuronale Netzwerk mit den bereitgestellten Trainingsdaten trainiert. Als Eingangsdaten des zu trainierenden Netzwerkes dienen die Eigenschaften des Operateurs und die Art des Eingriffs. Das neuronale Netzwerk wird trainiert, als Ausgangsdaten Konfigurationen des Therapieplatzes zu ermitteln, die von den jeweiligen Konfigurationen der Trainingsdaten für die entsprechenden Eingangsdaten möglichst wenig abweichen. Dies kann beispielsweise durch die Methode der Back-Propagation erreicht werden. Als Abweichung kann das Quadrat der Differenz des Ausgangswerts von dem entsprechenden Konfigurationswerte der Trainingsdaten bzw. der Quadratsumme bei mehrdimensionalen Ausgangswerten, wenn beispielsweise mehrere Stellglieder anzusteuern sind.

Auf vorteilhafte Weise ermöglicht ein neuronales Netzwerk das Modellieren komplexer Zusammenhänge, insbesondere, wenn keine mathematisch-, kinetisch- und Kinematik-funktionalen Zusammenhänge bekannt sind.

Weitere vorteilhafte Ausführungsformen sind zu den Unteransprüchen angegeben.

In einer denkbaren Ausführungsform des erfindungsgemäßen Verfahrens umfasst der Schritt des Ausgebens der Konfiguration ein Einstellen eines Stellgliedes gemäß einem Parameter der Konfiguration durch die Ausgabeeinheit. Mit anderen Worten, die Ausgabeeinheit weist ein Stellglied auf, mit dem die Steuerung eine Konfiguration des interventionellen Therapieplatzes direkt verändern kann. Das Stellglied kann beispielsweise eine Linearbewegung oder eine Drehung einer Komponente mit einem Motor bewirken, die eine Relativposition verändert, beispielsweise Höhe, Ausrichtung und/oder Neigung der Patientenliege, Sitzgelegenheit des Operateurs, eines Instrumentes und/oder einer Bildgebungsvorrichtung.

Auf vorteilhafte Weise kann durch das Stellglied eine wohldefinierte Positionierung erfolgen, ohne dass der Operateur von dem Eingriff abgelenkt wird.

In einer möglichen Ausführungsform des erfindungsgemäßen Verfahrens weist der operationelle Arbeitsplatz eine Bildgebungsvorrichtung auf. Bei der Bildgebungsvorrichtung kann es sich um einen Magnetresonanztomographen, einen C-Bogen, einen Computertomographen, einen Ultraschall-Bildgeber, ein Fluoroscop oder auch nur, wie nachfolgend ausgeführt, um eine Kamera, insbesondere eine 3D-Kamera handeln.

Eine Bildgebungsvorrichtung für einen Bereich der Intervention muss üblicherweise auch in dessen unmittelbaren Umgebung angeordnet sein, wie auch z.B. das Instrument und der Operateur. Eine optimale Abstimmung der Positionen ist daher für einen Erfolg des Eingriffs entscheidend. Gleichzeitig kann die Bildgebungseinrichtung auf vorteilhafte Weise auch wichtigen Input, beispielsweise über Eigenschaften des Operateurs durch eine Bilderfassung liefern.

In einer denkbaren Ausführungsform des erfindungsgemäßen Verfahrens weist die Vorrichtung zum Erfassen eine Kamera auf. Es kann sich dabei um eine 3D-Kamera handeln oder auch nur um eine einfach 2D-Kamera. Die Kamera kann einzelne Merkmale bzw. physiologische Eigenschaften des Operateurs erfassen, beispielsweise Größe oder Abmessungen einzelner Körperteile, die einen Einfluss auf eine Ausführung des Eingriffs haben. Es kann auch nur die Identität mittels der Kamera ermittelt werden, um entsprechende Eigenschaften aus einem Speicher oder einer Datenbank abzurufen. Denkbar ist aber ebenfalls, dass die Kamera durch Auswertung von Bildsequenzen auch Bewegungsabläufe erfasst und/oder deren Kinematik oder Biomechanik, die sich für jeden Operateur unterscheiden kann.

Denkbar ist es dabei auch, dass ein ganzer Ablauf des Eingriffs von der Kamera erfasst wird, sodass die Kinematik analysiert werden kann und die einzustellende Konfiguration für einen zukünftigen Eingriff angepasst werden kann, beispielsweise mittels eines neuronalen Netzwerks. Die Anwendung einer künstlichen Intelligenz bzw. eines neuronalen Netzwerkes ist beispielsweise auch nur zum Auswerten der Kameradaten beim Bestimmen einer Eigenschaft denkbar.

Auf vorteilhafte Weise erlaubt eine Kamera das Erfassen von Eigenschaften des Operateurs ohne zusätzliche Eingaben oder Unterbrechungen transparent für den Operateur.

In einer möglichen Ausführungsform des erfindungsgemäßen Verfahrens ist die Steuerung ausgebildet, die Konfiguration in Abhängigkeit von der physiologischen Eigenschaft, insbesondere einer Biomechanik des Operateurs bei dem vorzunehmenden Eingriff zu ermitteln. Denkbar ist beispielsweise, dass die Steuerung anhand eines biomechanischen Modells des Operateurs unter Zuhilfenahme der erfassten Eigenschaften, auch als digitaler Zwilling des Operateurs bezeichnet, eine Konfiguration des Therapieplatzes bzw. die relevanten Parameter optimiert, sodass eine physische Belastung des Operateurs beim Eingriff minimiert wird. Dies kann beispielsweise durch eine ergonomische Bewertung mit einer entsprechenden Software erfolgen.

Auf vorteilhafte Weise kann eine individuelle Anpassung des Therapieplatzes eine ergonomische Arbeitsweise und damit eine reduzierte physische Belastung sichergestellt werden.

In einer denkbaren Ausführungsform weist die Steuerung ein neuronales Netzwerk auf. Das neuronale Netz erhält eine Information zu dem Eingriff und eine Eigenschaft des Operateurs als Eingangssignal. In Abhängigkeit von der Form der Information können beispielsweise Teile bzw. Schichten des neuronalen Netzwerkes unterschiedlich ausgeführt sein. So ist es denkbar, dass bei einer Eingabe in Form einer Kamera eine zur Bilderkennung ausgeformte Netzwerkschicht eine oder mehrere Eigenschaften des Operateurs erkennt und in Sprachform weitergibt. Denkbar ist auch eine direkte Eingabe in Textform über eine Schnittstelle oder aus einer Datenbank. Die Konfiguration wird anschließend von der Steuerung mit Hilfe des neuronalen Netzwerks in Abhängigkeit von den Eingangsdaten ermittelt. Wenn von den Eingangsschichten die Eingangssignale in Textform angeliefert werden, kann das Ermitteln der Konfiguration beispielsweise durch ein neuronales Netzwerk in Form eines Large Language Models (LLM) erfolgen.

Auf vorteilhafte Weise bietet ein neuronales Netzwerk eine flexible Möglichkeit, anhand von Trainingsdaten auch analytisch nicht erfassbare Zusammenhänge zu berücksichtigen und dem Operateur eine verbesserte Arbeitsumgebung zu bieten.

In einer möglichen Ausführungsform des erfindungsgemäßen Verfahrens betrifft die Konfiguration eine Position und/oder Haltung des Operateurs relativ zu dem interventionellen Therapieplatz. Die Konfiguration kann beispielsweise Stellglieder der Ausgabeeinheit betreffen, die eine Sitzgelegenheit für den Operateur positionieren oder den Patiententisch relativ zu dem Operateur. Denkbar ist aber auch eine grafische Ausgabe auf einem Display, in Textform auf dem Display oder als Sprache oder durch visuelle Symbole.

Auf vorteilhafte Weise ermöglicht die Veränderung der Position des Operateurs einen weiteren Freiheitsgrad bei der Optimierung des Eingriffs.

In einer denkbaren Ausführungsform des erfindungsgemäßen Verfahrens zum Trainieren des neuronalen Netzwerks werden synthetische Ausgangsdaten zu Eingangsdaten ermittelt. Dazu wird die Konfiguration als Ausgangsparameter mit Hilfe eines biomechanischen Modells des Operateurs ermittelt, vorzugsweise unter Berücksichtigung ergonomischer Aspekte. Mit anderen Worten, für einen digitalen Zwilling des Operateurs und des Therapieplatzes werden Ausgangsparameter bzw. Konfigurationen des Therapieplatzes ermittelt, die mit einer Metrik einer Ergonomiebewertung eine optimale Lösung für den Operateur bereitstellt. Denkbar ist eine Optimierung mittels eines klassischen Optimierungsverfahrens wie LMS, aber auch ein mit Beispieldaten trainiertes neuronales Netzwerk.

In einer möglichen Ausführungsform des erfindungsgemäßen Verfahrens weisen die Trainingsdaten weiterhin einen Erfolgsparameter für die jeweiligen Kombinationen der Eingangsparameter und des Ausgangsparameters auf. Der Erfolgsparameter gibt ein Maß für die Konfiguration an, inwieweit sie für die Ausführung des Eingriffs zuträglich ist. Dieser Erfolgsparameter kann beispielsweise durch eine Bewertung eines Operateurs in vorhergehenden Eingriffen ermittelt werden oder durch ein Programm, das anhand eines biomechanischen Modells eine ergonomische Bewertung liefert.

Eine Nutzung von Erfolgsparametern als Gewichtung beschleunigt und verbessert eine Konvergenz zu einer verbesserten Konfiguration.

In einer denkbaren Ausführungsform des erfindungsgemäßen Verfahrens zum Trainieren des neuronalen Netzwerks weist das Verfahren weiterhin den Schritt auf, das neuronale Netz im Betrieb unter Nutzung einer erfassten Eigenschaft des Operateurs, eines vorgenommenen Eingriffs und einer dazu ermittelten Konfiguration in Abhängigkeit von einem dazu erfassten Erfolgsparameter anzupassen. Mit anderen Worten, das Training des neuronalen Netzwerks wird im laufenden Betrieb fortgesetzt, wobei die jeweils genutzten Eingangs- und Ausgangsparameter als zusätzliche Trainingsdaten genutzt werden.

Auf vorteilhafte Weise ist das neuronale Netzwerk so in der Lage, das Ergebnis weiter zu optimieren und sich veränderten Randbedingungen anzupassen, wie beispielsweise neue Operateure oder veränderte Eingriffe.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Es zeigen:
- Fig. 1: eine schematische Darstellung eines beispielhaften erfindungsgemäßen Therapieplatzes;
- Fig. 2: einen schematischen Ablaufplan einer Ausführungsform eines erfindungsgemäßen Verfahrens zur Konfiguration des Therapieplatzes.

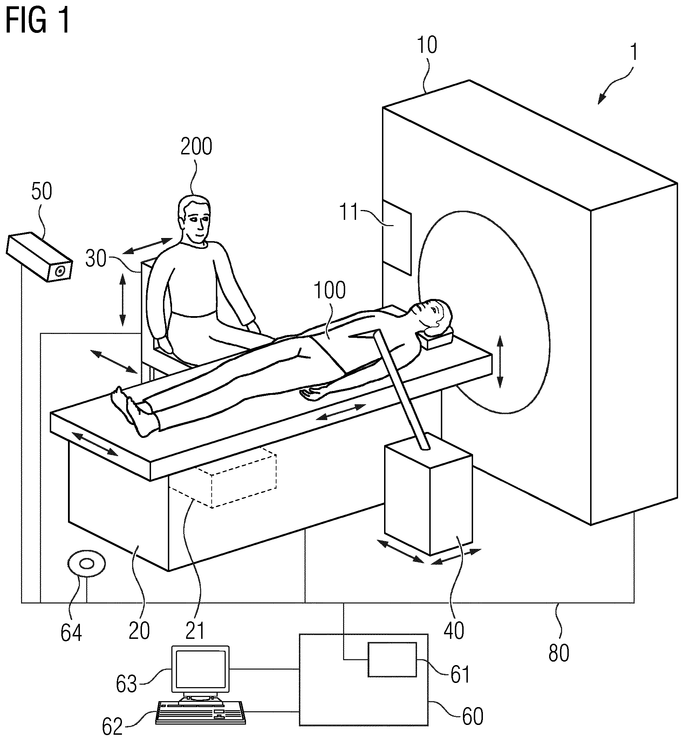

Fig. 1 zeigt eine schematische Darstellung eines beispielhaften erfindungsgemäßen interventionellen Therapieplatzes 1 zur Durchführung des erfindungsgemäßen Verfahrens.

Der Patient 100 ist auf einem Patiententisch 20 gelagert. Der Patiententisch 20 ist, wie beispielhaft durch die Richtungspfeile angedeutet, in mehreren Achsen durch eine Steuerung 60 mittels eines Antriebs 21 bewegbar.

Der Therapieplatz 1 kann weiterhin einen Sitz 30 für einen Operateur 200 aufweisen, der eine Intervention durchführt bzw. überwacht. Dazu benötigt der Operateur 200 einen Zugang oder zumindest eine entsprechende Ansicht des Patienten 100. In einer Ausführungsform ist es zu diesem Zweck denkbar, dass der Sitz 30 Antriebe bzw. Stellglieder aufweist, um eine Position und/oder Form des Sitzes zu verändern, um wie nachfolgend erläutert, dem Operateur 200 eine verbesserte Sitzposition für den Eingriff zu bieten. Denkbar sind z.B. eine Höhenverstellung, ein Verfahren einer Position des Sitzes 30 auf einem Untergrund des Therapieplatzes 1 relativ zu dem Patienten oder auch eine Verformung des Sitzes, um die Sitzposition des Operateurs 200 für einen besseren Zugriff auf den Patienten 100 anzupassen. Der mindestens eine Antrieb steht in Signalverbindung mit der Steuerung 60, um von dieser in eine vorbestimmte Position verbracht zu werden.

Der Therapieplatz 1 weist vorzugsweise eine Bildgebungsvorrichtung 10 auf, beispielsweise einen Magnetresonanztomographen oder einen Computertomographen, einen C-Bogen oder Ultraschallscanner, um eine Intervention im Inneren des Patienten 100 zu überwachen. Die Bildgebungsvorrichtung 10 kann dabei den Zugang des Operateurs 200 zu dem Patienten 100 behindern oder beschränken, sodass die Relativpositionen von Operateur 200, Patient 100 und Bildgebungsvorrichtung 10 einen Einfluss auf eine Dauer und Erfolg einer Intervention haben.

Der Therapieplatz 1 kann auch ein medizinisches Instrument 40 aufweisen, das den Eingriff unterstützt oder durchführt. Das medizinische Instrument 40 kann beispielsweise ein Roboter sein, der eine Biopsienadel oder einen Katheter führt, aber auch ein Laser, eine Bestrahlungseinrichtung oder ein anderes Instrument, mit dem am Patienten 100 ein Eingriff vorgenommen wird. Das medizinische Instrument 40 benötigt für den Eingriff einen Zugang zu dem Patienten 100 aus einer oder mehreren vorbestimmten Richtungen, sodass die relativen Positionen des Patienten 100 bzw. des Patiententischs 20, des Sitzes 30 bzw. des Operateurs 200, der Bildgebungsvorrichtung 10 und/oder des medizinischen Instruments 40 relativ zueinander Einfluss auf die Durchführung und den Erfolg eines Eingriffs haben. Vorzugsweise weist deshalb das medizinische Instrument 40 einen Antrieb bzw. Stellglied auf, der dessen Relativposition zu dem Therapieplatz 1 unter Kontrolle der Steuerung 60 verändern kann.

Es ist gemäß der Erfindung nicht erforderlich, dass alle Einheiten des Therapieplatzes 1 wie beschrieben jeweils einen Antrieb zur Veränderung der Position aufweisen. Beispielsweise kann eine der beteiligten Einheiten eine feste Position aufweisen und die anderen ihre relative Position dazu verändern. Insbesondere eine von den Abmessungen bzw. Gewicht wenig mobile Einheit wie die Bildgebungsvorrichtung 10 in Form eines Magnetresonanztomographen oder Computertomographen ist vorzugsweise ortsfest am Therapieplatz 1, während sich die anderen Einheiten relativ dazu positionieren bzw. in einer vorbestimmten Position ausrichten.

Auch ist es denkbar, dass eine Ausgabeeinheit der Steuerung 60 zum Verändern von Relativpositionen nicht ein Antrieb oder Stellglied ist, sondern insbesondere bezüglich der Position des Operateurs 200 auch eine Ausgabeeinheit mit Ausgabemöglichkeiten für Nachrichten an den Operateur 200 in visueller oder akustischer Form, die ihm Hinweise oder Anweisungen zu seiner Position geben. Stellvertretend für diese Art von Ausgabeeinheit ist ein Display 11 an der Bildgebungsvorrichtung 10 oder Display 62 an der Steuerung 60 angegeben, das in Signalverbindung mit der Steuerung 60 steht und von dieser gesteuert Ausgaben empfängt. Informationen können an einer Eingabevorrichtung 62, beispielsweise einer Tastatur oder einem grafischen Interface vorgenommen werden.

Der Therapieplatz 1 weist eine Vorrichtung zum Erfassen einer Eigenschaft des Operateurs 200 auf, hier beispielhaft eine Kamera 50, die den Operateur 200 an dem Therapieplatz 1 erfasst. Die Kamera kann beispielsweise eine 2D-Kamera sein. Mit dieser kann beispielsweise über eine Gesichtserkennung ein Operateur 200 erfasst, identifiziert und Informationen über den Operateur 200 aus einem Speicher oder einer Datenbank abgerufen werden. Zu diesen Informationen können auch physiologische Daten wie z.B. Größe oder biomechanische bzw. biokinetische Daten gehören. Denkbar ist auch, dass aus einem von der Kamera 50 aufgenommenen Bild bzw. Film derartige Daten bzw. Informationen von der Steuerung 60 oder einem neuronalen Netzwerk 61 aus dem Bild bzw. Film ermittelt werden. Die Kamera 50 kann auch Informationen zu einer Position der Einheiten des Therapieplatzes 1 wie Operateur 200, Patient 100, Bildgebungsvorrichtung 10 und/ oder medizinischem Instrument relativ zueinander erfassen, insbesondere, wenn es sich um eine 3D-Kamera handelt.

Die Vorrichtung zum Erfassen kann aber auch eine Eingabevorrichtung 62 sein, an der entsprechende Angaben eingegeben werden. Es ist auch denkbar, dass eine physiologische Eigenschaft des Operateurs 200 mit der Bildgebungseinrichtung 10 erfasst wird, die dann für eine spätere Verwendung gespeichert wird. Als eine erfindungsgemäße Eingabevorrichtung sind auch elektronische Schnittstellen wie RFID denkbar, mittels derer in Verbindung mit einem RFID-Tag der Operateur 200 identifiziert wird, oder mit der mittels damit verbundener Sensoren am Körper des Operateurs 200 entsprechende Daten erfasst und übermittelt werden.

Als Eingabevorrichtung kann auch ein Fußtaster 64 dienen. Mit dem Fußtaster 64 kann eine Auswahl erfolgen, vorzugsweise wird der Fußtaster 64 aber genutzt, um während des Eingriffs interaktiv beispielsweise eine Bilderfassung auszulösen oder ein medizinisches Instrument 40 zu aktivieren oder zu steuern. Dazu muss der Fußtaster 64 in geeigneter Relativposition zum Operateur 200 angeordnet sein und kann damit auch Teil der Konfiguration des Therapieplatzes 1 sein.

Die einzelnen Einheiten des Therapieplatzes 1 stehen in Signalverbindung, beispielsweise über ein Datennetzwerk 80, das auch in drahtloser Form wie WLAN oder Bluetooth denkbar ist.

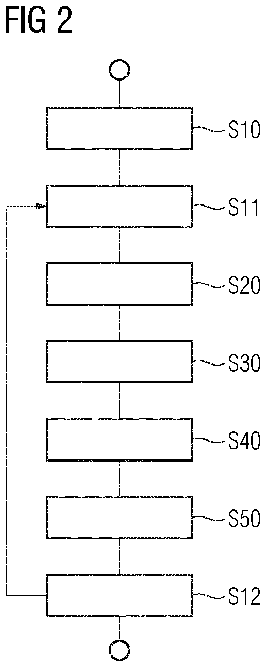

Fig. 2 zeigt einen schematischen Ablaufplan einer Ausführungsform eines erfindungsgemäßen Verfahrens zur Konfiguration des Therapieplatzes 1.

In einem Schritt S20 wird mindestens eine Eigenschaft des Operateurs 200 mit der Vorrichtung zum Erfassen durch die Steuerung 60 erfasst. Beispielsweise kann die Kamera 50 als Vorrichtung zum Erfassen den Operateur abbilden und die Steuerung 60 dann aus dem Bild die Identität oder eine Abmessung des Körpers des Operateurs 200 ermitteln. Es ist auch denkbar, dass die Kamera 50 in einer Abfolge von Bildern oder einem Film kinematische Eigenschaften erfasst bzw. einen digitalen Twin des Operateurs in Bezug auf Bewegungsabläufe und/oder Ergonomie erfasst. Dies ist insbesondere für eine 3D-Kamera möglich.

Es ist aber auch denkbar, dass eine physiologische Eigenschaft des Operateurs 200 indirekt erfasst wird, indem zunächst eine Identität des Operateurs 200 mit einem Kennzeichen erfasst wird, z.B. über ein drahtloses Token bzw. Tag, und entsprechende physiologische Eigenschaften anhand gespeicherter Daten zu dem Operateur 200 aus einem Speicher oder einer Datenbank abgerufen werden. Die physiologischen Eigenschaften können dabei auch vorab mittels der Bildgebungsvorrichtung 10 erfasst worden sein.

Es ist in Schritt S20 Erfassen der Eigenschaften insbesondere auch denkbar, dass die physiologischen Eigenschaften mittels eines trainierten neuronalen Netzwerkes 61 aus den erfassten Abbildungen der Kamera 50 ermittelt werden.

In einem weiteren Schritt S30 erfasst die Steuerung 60 einen vorzunehmenden Eingriff über eine Schnittstelle. Die Schnittstelle kann eine Eingabevorrichtung 62 wie beispielsweise eine Tastatur oder eine grafische Schnittstelle sein, die eine Eingabe eines Nutzers zu dem Eingriff erfasst. Denkbar ist auch eine Information über ein Datennetzwerk von einem Kliniksystem, mit dem Eingriffe geplant und/oder Patientendaten verwaltet werden.

In einem Schritt S40 ermittelt die Steuerung 60 eine Konfiguration für den interventionellen Therapieplatz 1 in Abhängigkeit von dem vorzunehmenden Eingriff und mindestens einer Eigenschaft des Operateurs 200. Mit anderen Worten, die Steuerung 60 sucht vorzugsweise eine Konfiguration des Therapieplatzes 1, die für den erfassten Eingriff und den Operateur 200 bzw. für einen Operateur 200 mit der erfassten physiologischen Eigenschaft den Ablauf des Eingriffs verbessert, insbesondere für den Erfolg des Eingriffs besonders erfolgsversprechend sind und/oder für den Operateur 200 ergonomisch vorteilhaft sind, d.h. wenig physisch belastend. Die Auswahl kann anhand einer Datenbank mit gespeicherten Eingriffen, Operateuren 200 bzw. deren Eigenschaften und Konfiguration erfolgen, wobei für die entsprechenden Kombinationen bzw. Tupel aus Eingriff, Eigenschaft des Operateurs 200 und Konfiguration Wertungen erfasst sind.

Denkbar ist auch ein neuronales Netzwerk 61, beispielsweise ein Large-Language-Model, das mit dem nachfolgend beschriebenen Trainingsverfahren darauf trainiert ist, aus den Eingangsparametern, d.h. für den auszuführenden Eingriff und der mindestens einen physiologischen Eigenschaft des Operateurs 200 als Ausgabe, eine Konfiguration zu ermitteln.

Beispielsweise kann ein durchzuführender Eingriff eine Nadelbiopsie sein, die mittels eines Magnetresonanztomographen als Bildgebungsvorrichtung 10 überwacht werden soll. Der Patient 100 ist dabei mit dem Organ, für das eine Biopsie erfolgen soll, in einem Isozentrum, d.h. einem Bereich der höchsten Magnetfeldhomogenität, des Magnetresonanztomographen zu lagern. Die Konfiguration des Therapieplatzes hängt dabei beispielsweise von Körpergröße und Armlänge des Operateurs 200 ab. Ist der Operateur relativ klein, muss der Operateur 200 relativ hoch zu dem Patienten 100 bzw. dem Magnetresonanztomographen stehen oder sitzen, sodass er sich weit in einen Patiententunnel des Magnetresonanztomographen in Richtung des Isozentrums vorbeugen kann, um dieses zu erreichen. Ist die Armlänge überdurchschnittlich, so kann er das Isozentrum bereits erreichen, ohne dass er sich so weit überbeugt und so die Wirbelsäule belastet. Ist er auch noch überdurchschnittlich groß, so kann es von Vorteil sein, wenn der Patient 100 bzw. der Magnetresonanztomograph relativ zu dem Operateur 200 höher gelagert ist, sodass er sich nicht so weit hinunterbücken muss. Die Höhendifferenz kann dabei verändert werden, indem der Magnetresonanztomograph angehoben oder abgesenkt wird, oder/und der Operateur 200 in entgegengesetzter Richtung, mittels eines verstellbaren Sitzes 30 oder einer Standplattform. Auch ist eine Unterstützung der Position durch ein Exoskelett als Teil der Ausgabeeinheit denkbar.

Dabei ist das erläuterte Beispiel nur zur bildhaften Erläuterung gedacht. Es sind im Rahmen der Erfindung noch viele andere Kombinationen aus physiologischen Parametern, Eingriffen und Bildgebungsvorrichtung denkbar, bei denen die Konfiguration in vergleichbarer Weise angepasst wird.

In einem weiteren Schritt S50 wird die ermittelte Konfiguration über die Ausgabeeinheit ausgegeben. Ist die Ausgabeeinheit ein Stellglied, beispielsweise ein Antrieb 21 in Patientenliege 20, stellt die Steuerung 60 über eine Signalverbindung, beispielsweise das Datennetzwerk 80, eine ermittelte Position des Stellgliedes ein. Bei der Patientenliege 20 kann das beispielsweise eine Höhe der Liegefläche oder Position entlang der Längsachse eines Patiententunnels eines Magnetresonanztomographen sein. Für einen Sitz 30 können dies beispielsweise eine Position relativ zu dem Patiententisch 20 über ein Fahrwerk als Stellglied sein, eine Höhe, Neigung oder Ausrichtung der Sitzfläche. Die Bildgebungsvorrichtung 10 und/oder das medizinische Instrument 40 können ebenfalls eine oder mehrere Stellglieder als Teil der Ausgabeeinheit aufweisen, die eine Ausrichtung oder Position der Einheiten relativ zueinander und/oder dem Patienten 100 und/oder dem Operateur 200 verändern. Insbesondere, wenn die Konfiguration aber eine Haltung oder Position des Operateurs 200 betrifft, ist es auch denkbar, dass die Ausgabeeinheit eine visuelle oder akustische Information an den Operateur 200 ausgibt, wie er sich positionieren soll. Dies kann z.B. eine bildliche Darstellung auf dem Display 11 sein, aber in Zusammenarbeit mit der Kamera 50 und der Steuerung 60 auch ein Erfassen der Ist-Position, ermitteln einer Abweichung von der Soll-Position und Ausgabe einer beispielsweise akustischen Anweisung, wie die Soll-Position ausgehend von der Ist-Position zu erreichen ist.

Der Schritt S50 kann auch eine Mehrzahl und einen Mix der beschriebenen Ausgaben umfassen, wenn die Konfiguration aus einer entsprechenden Mehrzahl an Ausgangsparameter besteht.

Erfolgt das Ermitteln der Konfiguration in Schritt S40 mit einer künstlichen Intelligenz (KI) bzw. einem neuronalen Netzwerk 61, so ist es erforderlich, die KI bzw. das neuronale Netzwerk 61 in einem entsprechenden Verfahren zu trainieren.

Bei dem erfindungsgemäßen Verfahren zum Trainieren werden in einem Schritt S10 eine Vielzahl von Tupeln aus Eigenschaften von Operateuren 200, von Eingriffen und von zugeordneten Konfigurationen als Trainingsdaten bereitgestellt. Ein derartiges Tupel kann beispielsweise aus geeigneten Aufzeichnungen eines bereits erfolgten Eingriffs bereitgestellt werden. Die geeigneten Aufzeichnungen stellen jeweils Informationen zu dem Eingriff, Eigenschaften des Operateurs 200 und eine verwendete Konfiguration bereit, die zu einem entsprechenden Tupel zusammengefasst werden können.

Denkbar ist es auch, dass derartige Trainingsdaten durch eine Simulation einzelner Eingriffe erzeugt werden, wobei für die jeweiligen Operateure 200 mit den entsprechenden Eigenschaften biomechanische Modelle mit entsprechend dimensionierten Eingangsparametern genutzt werden. Ein derartiges biomechanisches Modell berücksichtigt dabei vorzugsweise biomechanisch mögliche Bewegungen des Operateurs zur Durchführung und dabei entstehende Belastungen für Muskeln und Gelenke. Daraus ist dann mit den üblichen Optimierungsverfahren eine Konfiguration mit minimaler Belastung ermittelbar, beispielsweise durch ein iteratives Verfahren oder wiederum eine entsprechend trainierte künstliche Intelligenz bzw. neuronales Netzwerk.

In einem Schritt S11 Trainieren des neuronalen Netzwerkes 61 werden die Tupel mit den Eigenschaften des Operateurs 200 und die Art des Eingriffs als Eingangsparameter des neuronalen Netzwerkes 61 diesem zugeführt. Die künstliche Intelligenz bzw. das neuronale Netzwerk 61 erzeugt dazu anhand seines internen Parametersatzes eine Konfiguration als Ausgangswert zu diesen Eingangsparametern. Das neuronale Netzwerk 61 wird dabei durch Anpassen seines internen Parametersatzes dahingehend trainiert, eine Abweichung einer Konfiguration als Ausgangswert von einer Konfiguration in den Trainingsdaten bei den Eingangsparametern der Trainingsdaten zu minimieren. Dies kann bei einem neuronalen Netzwerk beispielsweise durch das Verfahren der Back-Propagation erreicht werden.

In einer bevorzugten Ausführungsform des Verfahrens zum Trainieren weisen die Trainingsdaten in dem Tupel noch mindestens einen Gewichtungsparameter auf, der beispielsweise ein Maß für einen Erfolg des Eingriffs und/oder Belastung des Operateurs mit den jeweiligen Eingangsparametern des Tupels bei der jeweiligen Konfiguration des interventionellen Therapieplatzes angibt. Der Gewichtungsparameter kann beispielsweise durch eine Bewertung des Operateurs oder eine Metrik des biomechanischen Modells für eine Belastung des Operateurs gewonnen werden.

In dem Schritt S11 Trainieren des neuronalen Netzwerks 61 erfolgt dann die Anpassung des neuronalen Netzwerks 61 in Abhängigkeit von diesem Gewichtungsparameter. Beispielsweise werden die internen Parameter stärker verändert, wenn der Gewichtungsparameter eine geringe Belastung und/oder einen hohen Erfolg des Eingriffs mit den Eingangsparametern und der Konfiguration angibt.

In einer Ausführungsform des erfindungsgemäßen Verfahrens zum Trainieren ist es denkbar, dass es zusammen mit dem Verfahren zum Konfigurieren angewendet wird. Dabei werden jeweils die im Verfahren zur Konfiguration verwendeten Eingangsdaten und die ermittelte Konfiguration von der Steuerung 60 in einem Schritt S12 zu einem Tupel an Trainingsdaten zusammengefasst. Vorzugsweise wird am Abschluss des Eingriffs noch eine Bewertung in Form eines Gewichtungsparameters erfasst.

Dieses Tupel wird anschließend gemäß dem Schritt S11 des Verfahrens zum Trainieren zur Modifikation bzw. Adaptierung des neuronalen Netzwerks 61 angewendet.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zur Konfiguration eines interventionellen Therapieplatzes (1) an einen Operateur (200) zur Durchführung eines Eingriffs, wobei der interventionelle Therapieplatz (1) eine Steuerung (60), eine Schnittstelle zur Eingabe einer Information über den Eingriff, eine Vorrichtung zum Erfassen eines Operateurs (200) und mindestens eine Ausgabeeinheit zur Anpassung einer Konfiguration des interventionellen Therapieplatzes (1) aufweist, wobei das Verfahren die Schritte aufweist:
(S20) Erfassen mindestens einer Eigenschaft des Operateurs (200) mit der Vorrichtung zum Erfassen durch die Steuerung (60) ;
(S30) Erfassen eines vorzunehmenden Eingriffs über die Schnittstelle durch die Steuerung (60);
(S40) Ermitteln einer Konfiguration für den interventionellen Therapieplatz (1) in Abhängigkeit von dem vorzunehmenden Eingriff und mindestens einer Eigenschaft des Operateurs (200) durch die Steuerung (60);
(S50) Ausgeben der ermittelten Konfiguration über die Ausgabeeinheit.

2. Verfahren nach Anspruch 1, wobei der Schritt (S50) des Ausgebens der Konfiguration ein Einstellen eines Stellgliedes gemäß einem Parameter der Konfiguration durch die Ausgabeeinheit umfasst.

3. Verfahren nach Anspruch 1 oder 2, wobei der interventionelle Therapieplatz (1) eine Bildgebungsvorrichtung (10), insbesondere einen Magnetresonanztomographen aufweist.

4. Verfahren nach einem der vorhergehenden Ansprüche, wobei die Vorrichtung zum Erfassen eine Kamera (50) aufweist, und die erfasste Eigenschaft des Operateurs (200) eine physiologische Eigenschaft ist, die einen Einfluss auf eine Ausführung des Eingriffs hat.

5. Verfahren nach Anspruch 3, wobei die Steuerung (60) die Konfiguration in Abhängigkeit von der physiologischen Eigenschaft des Operateurs (200) bei dem vorzunehmenden Eingriff ermittelt.

6. Verfahren nach Anspruch 5, wobei die Steuerung (60) ein neuronales Netzwerk (61) aufweist und die Konfiguration von der Steuerung (60) mit Hilfe des neuronalen Netzwerks (61) ermittelt.

7. Verfahren nach einem der vorhergehenden Ansprüche, wobei die Konfiguration eine Position und/oder Haltung des Operateurs (200) relativ zu dem interventionellen Therapieplatz (1) betrifft.

8. Medizinische Bildgebungsvorrichtung, wobei die Bildgebungsvorrichtung (10) eine Steuerung (60), eine Schnittstelle zur Eingabe einer Information über einen Eingriff, eine Vorrichtung zum Erfassen eines Operateurs (200) und mindestens eine Ausgabeeinheit zur Anpassung einer Konfiguration des Therapieplatzes aufweist, wobei die Steuerung (60) ausgebildet ist, das Verfahren nach einem der Ansprüche 1 bis 7 auszuführen.

9. Verfahren zum Trainieren eines neuronalen Netzwerkes (61), wobei das Verfahren die Schritte aufweist:
(S10) Bereitstellen einer Vielzahl von Tupeln aus Eigenschaften von Operateuren (200), von Eingriffen und von zugeordneten Konfigurationen als Trainingsdaten;
(S11) Trainieren des neuronalen Netzwerkes (61), wobei die Eigenschaften des Operateurs (200) und die Art des Eingriffs Eingangsparameter des neuronalen Netzwerkes (61) sind und eine zugehörige Konfiguration ein Ausgangswert, wobei das neuronale Netzwerk (61) trainiert wird, eine Abweichung einer Konfiguration als Ausgangswert von einer Konfiguration in den Trainingsdaten bei den Eingangsparametern der Trainingsdaten zu minimieren.

10. Verfahren zum Trainieren nach Anspruch 9, wobei bei dem Bereitstellen der Trainingsdaten eine Konfiguration als Ausgangsparameter zu vorbestimmten Paaren aus Eigenschaft und Eingriff unter Anwendung eines biomechanischen Modells für den Operateur ermittelt wird.

11. Verfahren zum Trainieren nach Anspruch 9 oder 10, wobei die Trainingsdaten weiterhin einen Erfolgsparameter für die jeweiligen Kombinationen der Eingangsparameter und des Ausgangsparameters aufweisen und das neuronale Netzwerk (61) in Abhängigkeit von dem Erfolgsparameter trainiert wird.

12. Verfahren zum Trainieren nach Anspruch 11, wobei das neuronale Netz (61) in einem weiteren Schritt im Betrieb unter Nutzung einer erfassten Eigenschaft des Operateurs (200), eines vorgenommenen Eingriffs und einer dazu ermittelten Konfiguration in Abhängigkeit von einem dazu erfassten Erfolgsparameter angepasst wird.
